# EUROPEAN PATENT APPLICATION

(11) **EP 1 908 775 A1**
(43) Date of publication of application: **09.04.2008**
(21) Application number: 06021053.1
(22) Date of filing: 06.10.2006
(51) Int. Cl.: C07K 14/435, C12N 15/00

(54) **Fluorescent proteins from the Ctenophora phylum and methods of use thereof**

(71) Applicant: AXXAM S.p.A., 20132 Milano (IT)
(72) Inventor: Mastroianni, Nadia, 20132 Milano (IT); Corazza, Sabrina, 20132 Milano (IT); Lohmer, Stefan, 20132 Milano (IT)
(74) Representative: Banfi, Paolo

(57) **Abstract**

The invention discloses fluorescent proteins isolated from organisms of the *Ctenophora* phylum, derivatives, homologues and mutants thereof as well as the use thereof in reporter gene technology and in cell-based and cell-free assays, particularly for protein labelling, in the study of protein interactions and location, in diagnostics and medical imaging.

SEQUENCE LISTING

<110>  AXXAM s.r.l.

<120>  Fluorescent proteins from the Ctenophora phylum and methods of
       use thereof

<130>  1725eur

<160>  6

<170>  PatentIn version 3.3

<210>  1
<211>  262
<212>  PRT
<213>  Unknown

<220>
<223>  Haeckelia beehleri

<400>  1

Met Glu Phe Glu Pro Glu Phe Phe Asn Lys Pro Val Pro Leu Glu Met
1               5                   10                  15

Thr Leu Arg Gly Cys Ile Asn Gly Lys Glu Phe Met Ile Phe Gly Lys
            20                  25                  30

Gly Glu Gly Asp Ala Ser Lys Gly Asn Ile Lys Gly Lys Trp Val Leu
        35                  40                  45

Ser His Ser Glu Asp Gly Lys Cys Pro Met Ser Trp Ala Val Leu Ala
    50                  55                  60

Pro Thr Phe Ala Tyr Gly Phe Lys Val Phe Ala Lys Tyr Pro Lys Asp
65                  70                  75                  80

Phe Ala His Phe Trp Gln Asp Cys Met Pro Val Gly Tyr Ser Glu Arg
                85                  90                  95

Arg Ile Thr Arg Phe Gly Arg Leu Ser Gly Asn Asp Asp Ile Glu Gln
            100                 105                 110

Glu Gly Ile Met Asn Thr Tyr His Glu Val Gln Met Arg Glu Arg Met
        115                 120                 125

Val Gly Asp Glu Ile Thr Trp Ile Val Glu Ser Arg Val Lys Leu Asp
    130                 135                 140

Ala Thr Ile Asn Glu Asn Ser Pro Ile Leu Met Asn Asp Gly Leu Ser
145                 150                 155                 160

Glu Tyr Arg Pro Asn Leu Glu Arg Thr Val Ser Phe Glu Asp Gly Leu
                165                 170                 175

Lys Asn Tyr Ser Gln Phe Phe Tyr Pro Ile Lys Asp Cys Glu Thr Asn
            180                 185                 190

Asp Tyr Ile Ile Ala Asn Gln Met Thr His Glu Arg Pro Leu Ser Lys
        195                 200                 205

Cys Asn Lys Pro Gly Arg Leu Pro Pro Ser His Phe Lys Arg Thr Asp
    210                 215                 220

Leu Glu Gln Trp Lys Asp Ser Lys Glu Asp Lys Asp His Ile Val Gln
225                 230                 235                 240

Glu Glu Ile Thr Ala Phe Leu Leu Gln Ala Gln Asp Lys Asp Leu Gln
                245                 250                 255

Ser Leu Gly Ile Gly Met
            260

<210>  2
<211>  1244
<212>  DNA
<213>  Unknown

<220>
<223>  Haeckelia beehleri

<400>  2
ggccattacg gccggggagt tttaaactta cactaactac tatacaaatt acaatttgag      60

aaacttacca tggagtttga gcctgaattc ttcaataaac ctgtacctct ggagatgact     120

cttcgtggat gtatcaacgg aaaagagttc atgatcttcg gtaagggtga aggggatgca     180

agtaaaggaa atataaaggg aaaatgggtc ctttctcatt ctgaagacgg aaaatgtcct     240

atgtcttggg cagttttggc tccaacattt gcctatggat tcaaagtgtt tgcaaagtac     300

ccaaaagatt ttgcgcactt ttggcaagat tgcatgcccg tgggatactc agaaaggaga     360

atcacaagat ttggacgtct cagtggcaat gacgacattg aacaagaagg aataatgaac     420

acttatcacg aggttcaaat gcgtgaacgt atggttgggg atgaaataac atggattgtt     480

gagagcagag taaaactaga tgcaactatc aatgagaaca gtcccatttt gatgaatgat     540

gggctatctg aataccgtcc aaatttagag cgaactgtat cctttgaaga tggtttaaag     600

aactacagtc aatttttcta tcctatcaaa gattgtgaga cgaacgatta cataatagca 660

aaccaaatga ctcacgaaag accgctgagc aaatgcaata aaccaggacg actcccgcca 720

tcccacttta aacgtactga ccttgagcaa tggaaggatt cgaaagagga caaagatcat 780

atcgttcaag aagagatcac tgcattcctc cttcaagcac aagacaagga tttacaaagc 840

cttggcattg gaatgtagat tgtagaaata taaatattgc aaagtatatt ctgagtaatg 900

aatgatccaa acactgtatg gaagaatata aataatgatt aataaattag agcaaataca 960

gttcaataat aaaccaatat tttaatattt tatctttctg aaggttttat atttaatcat 1020

aattttcaag tttgctcaaa ttgtcaatat gaaggaccta tttagtaaat ataaaatatt 1080

aatgagatca ggtaaatctt tctgaatttt ttggctaaaa tcctagtagt ataaggcaag 1140

ctacagtctc aaaccaaaaa ataatgacgt tgtaatatgt tattttactc ttttttgaat 1200

tatgaccatt ggtgaaaaaa aaaaaaaaaa aaaaaaaaaa acat 1244

<210> 3
<211> 789
<212> DNA
<213> Unknown

<220>
<223> Haeckelia beehleri

<400> 3
atggagtttg agcctgaatt cttcaataaa cctgtacctc tggagatgac tcttcgtgga 60

tgtatcaacg gaaaagagtt catgatcttc ggtaagggtg aaggggatgc aagtaaagga 120

aatataaagg gaaaatgggt cctttctcat tctgaagacg gaaaatgtcc tatgtcttgg 180

gcagttttgg ctccaacatt tgcctatgga ttcaaagtgt ttgcaaagta cccaaaagat 240

tttgcgcact tttggcaaga ttgcatgccc gtgggatact cagaaaggag aatcacaaga 300

tttggacgtc tcagtggcaa tgacgacatt gaacaagaag gaataatgaa cacttatcac 360

gaggttcaaa tgcgtgaacg tatggttggg gatgaaataa catggattgt tgagagcaga 420

gtaaaactag atgcaactat caatgagaac agtcccattt tgatgaatga tgggctatct 480

gaataccgtc caaatttaga gcgaactgta tcctttgaag atggtttaaa gaactacagt 540

caatttttct atcctatcaa agattgtgag acgaacgatt acataatagc aaaccaaatg 600

actcacgaaa gaccgctgag caaatgcaat aaaccaggac gactcccgcc atcccacttt 660

aaacgtactg accttgagca atggaaggat tcgaaagagg acaaagatca tatcgttcaa 720

gaagagatca ctgcattcct ccttcaagca caagacaagg atttacaaag ccttggcatt 780

ggaatgtag 789

<210> 4

<211>  792
<212>  DNA
<213>  Unknown

<220>
<223>  Haeckelia beehleri sequence with modified codon usage

<400>  4
atggaattcg agcccgagtt cttcaacaag cccgtgcccc tggaaatgac cctgcggggc      60

tgcatcaacg gcaaagaatt catgatcttc ggcaagggcg agggcgacgc cagcaagggc     120

aacatcaagg gcaagtgggt gctgtcccac agcgaggacg gcaagtgccc catgagctgg     180

gccgtgctgg cccccacctt cgcctacggc ttcaaggtgt tcgccaagta ccccaaggac     240

ttcgcccact tctggcagga ctgcatgccc gtgggctaca gcgagcggcg gatcacccgg     300

ttcggcagac tgagcggcaa cgacgacatc gagcaggaag gcatcatgaa cacctaccac     360

gaggtgcaga tgcgggagcg gatggtcggc gacgagatca cctggatcgt ggagagccgg     420

gtgaagctgg acgccaccat caacgagaac agccccatcc tgatgaacga cggcctgagc     480

gagtaccggc ccaacctgga acggaccgtg agcttcgagg acggcctgaa gaactacagc     540

cagttcttct accccatcaa ggactgcgag accaacgact acatcatcgc caaccagatg     600

acccacgagc ggcccctgag caagtgcaac aagcctggcc ggctgccccc cagccacttc     660

aagcggaccg acctggaaca gtggaaggac agcaaagagg acaaggacca cattgtgcag     720

gaagagatca ccgccttcct gctgcaagcc caggacaagg atctgcagag cctgggcatc     780

ggcatgtgat ga                                                         792


<210>  5
<211>  35
<212>  DNA
<213>  Unknown

<220>
<223>  artificial oligonucleotide

<400>  5
acgcgtcgac gtaagcagtg gtatcaacgc agagt                                 35


<210>  6
<211>  40
<212>  DNA
<213>  Unknown

<220>
<223>  artificial oligonucleotide

<400>  6
aaggaagagc ggccgcattc tagaggccga ggcggccgac                            40

## Description

The present invention relates to fluorescent proteins isolated from organisms of the *Ctenophora* phylum. More specifically, the invention provides nucleic acids and proteins isolated from organisms of the *Haeckeliidae* family and their derivatives, homologues and mutants. The fluorescent proteins of the invention present advantageous properties such as high stability at 37°C, high water solubility and low bulkiness, all characteristics which make their use in reporter gene technology and in cell-based assays particularly convenient. The fluorescent proteins of the invention and their encoding polynucleotides are also useful for protein labelling, in the study of protein interactions, in diagnostics and medical imaging.

### BACKGROUND OF THE INVENTION

The enormous variety of colors and fluorescent shades displayed by living organisms are determined by chromoproteins and low molecular weight pigments. Chromoproteins typically require a prosthetic group, such as a small nonpeptide molecule or metal ion, which binds to the protein and is essential for the chromogenic properties of the protein (Herring, P J, 1985).

Fluorescent proteins (FP) have been described and characterized from many marine invertebrates. Many members of the coelenterate phylum and the cnidarian sub-phylum contain fluorescent proteins (Shimomura O et al., 1962; Morin JG and Hastings JW, 1971; Wampler JE et al., 1971; Cormier MJ et al. 1973, 1974; Morin JG et al., 1974). The two best characterized and most well known fluorescent proteins are *Aequorea victoria* and *Renilla reniformis* GFPs (Tsien RY et al. 1998).

In 1955 Davenport et al. reported that the photogenic cells of the bioluminescent jellyfish *Aequorea victoria* fluoresced green when animals were irradiated with long-wave ultraviolet light. The first light-emitting proteins, a photoprotein and a green fluorescent protein (GFP), were isolated in 1962 from the jellyfish *Aequorea victoria* (Johnson FH et al. 1962, Shimomura O et al. 1962). The photoprotein was named Aequorin and is activated by calcium, which binds to the three EF-hand structures, typical of calcium binding proteins, present within the protein. In the presence of calcium Aequorin undergoes a conformational change, leading to the oxidation of bound coelenterazine with the subsequent release of CO₂ and a flash of light. *Aequorea victoria* GFP is part of the jellyfish bioluminescent system and plays a role of a secondary emitter, transforming the blue light from the aequorin photoprotein into green light. In fact, GFP serves as an energy-transfer acceptor by absorption of blue light at λ max = 470 nm from Ca²⁺-activated Aequorin and thereupon emits green light with emission at λ max = 508 nm (Morise H, 1974; Johnson FH et al, 1962).

In 1978, Prendergast FG et al. determined the molecular weight of monomeric GFP to be around 27 kDa and then in 1992 the cloning of the wild-type gene was accomplished by Prasher DC et al. The gene encodes a 238 amino acid protein which was expressed in different expression systems (Chalfie M et al., 1994) and can be used in practically any organism due to the unique ability of GFP to form a fluorophore by itself. The heterologous expression of GFP proved that its maturation and formation of its fluorophore was independent on factors such as enzymes or other proteins deriving from *Aequorea victoria.* In fact, it was revealed that the gene's protein sequence intrinsically encodes a strong fluorophore which does not require external substrates or co-factors except for molecular oxygen (Prasher DC et al., 1992; Cody CW et al., 1993; Heim R et al., 1994).

The crystal structure of GFP was independently solved in 1996 by Ormö M et al and Yang F et al. On the basis of these structures a new protein fold was discovered, which Yang et al. have named the *β*-Can structure. It consists of 11 antiparallel β-sheets which form a β-barrel and this barrel is crossed by an α-helix. A tripeptide sequence (Ser 65, Tyr 66, Gly 67) is located almost in the middle of the helix, which serves by an autocatalytic reaction as the backbone for the fluorophore structure.

Many experiments and their conclusions paved the way for GFPs to become an important tool in molecular and cellular biology as reporters of gene expression, fusion tags to monitor protein localization within living cells, tracers of cell lineage, partners for fluorescence resonance energy transfer (FRET) and biosensors (Tsien RY, 1998; Kendall JM, et al. 1998).

Despite its remarkable usefulness, wild-type GFP exhibits some limitations, such as problems with expression level, photosensitivity, low brightness, insolubility and the tendency to dimerize at high concentrations. To overcome these reported disadvantages, efforts were made to improve the wild-type GFP by mutagenesis in order to obtain mutants which are more soluble, photo-resistant, brighter and which have a shifted wavelength.

A number of new fluorescent proteins (FPs) have since been isolated from natural sources. More than 100 GFP-like green, yellow and red fluorescent FPs and nonfluorescent chromoproteins (CPs) have been cloned so far from a variety of coelenterates in the classes of Hydrozoa such as *Aequorea, Obelia* and *Phialidium* and Anthozoa such as *Renilla, Anemonia sulcata* and *Discosoma* (Wiedenmann J, et al. 2000; Matz MV, et al. 1999; Fradkov AF et al, 2000; Labas YA et al, 2002; Lukyanov KA et al, 2000; Gurskaya NG et al, 2001). Despite a modest degree of amino acid sequence identity, all GFP-like proteins are likely to share the β-Can structure, and sequence alignments show furthermore that amino acids which are a part of the chromophore, such as Tyr 66, Gly 67 and others which interact with the chromophore, such as Arg 96 and Glu 222 (numbering accordingly GFP) are conserved in the FPs.

One great disadvantage of the Anthozoan derived GFP-like proteins is their propensity to oligomerize (Verkhusha VV et al., 2004). Oligomerization does not narrow the usage of these proteins as reporter genes or biomarkers, but hampers their use in fusion protein applications, because fusions with them form bulky products which could interfere in the translocation of the target protein, especially in cases where the target protein itself tends to form oligomers. Another aspect is that some proteins are activated by oligomerization and fusing the protein with these FPs could lead to constitutively active target proteins which could harm the host cell. For all these reasons it is important and necessary to find or create monomeric FPs which minimally affect a target protein.

Cloning and characterization of fluorescent proteins from a different class than the Anthozoa and Hydrozoa opens a new perspective and allows for the possibility of obtaining FPs with improved features.

### DISCLOSURE OF THE INVENTION

In a first aspect, the invention provides an isolated nucleic acid molecule encoding a fluorescent protein or polypeptide which:
(a) has an amino acid sequence of SEQ ID NO: 1; or
(b) displays at least 80%, preferably at least 90%, more preferably at least 95% sequence identity to SEQ ID NO: 1; or
(c) is a fragment of a protein according to (a) or (b), said fragment being water-soluble and retaining the capability of emitting green light upon excitation with light in the range of λ 460-495 nm.

In a preferred embodiment, the isolated nucleic acid molecule according to the invention is selected from the group consisting of:
(a) a nucleic acid comprising a nucleotide sequence SEQ ID NO:2 or SEQ ID NO:3 or a complementary strand thereof;
(b) a nucleic acid differing from SEQ ID NO:2 or SEQ ID NO:3 due to the degeneracy of genetic code;
(c) a nucleic acid that hybridizes under stringent conditions to the nucleic acid of (a) or (b).

In a further preferred embodiment, the nucleic acid molecule according to the invention is isolated from organisms which taxonomically belong to *Ctenophora* phylum, *Typhlocoela* class, *Cydippida* order, *Haeckeliidae* family, *Haeckelia* genus and *Haeckelia beehleri* species.

The invention further comprises fragments of the nucleic acids herein disclosed and oligonucleotides hybridizing thereto. The fragments of a cDNA encoding a fluorescent protein according to the invention, preferably the cDNA set forth in SEQ ID NO: 3, as well as the oligonucleotides may be used as hybridization probes against a cDNA library of a target organism of interest. Nucleic acids having sequence similarity may be detected by hybridization under low stringency conditions, for example at 50°C and 6xSSC (0.9 M sodium chloride/ 0.09 M sodium citrate) and remain bound when subjected to washing at 55°C in 1xSSC (0.15 M sodium chloride/0.15 M sodium citrate), whereas sequence identity may be determined by hybridization under stringent conditions, for example at 50°C or higher and 0.1xSSC (15 mM sodium chloride/1. 5 mM sodium citrate). If used as primers, the oligonucleotides may hybridize to different regions of the target molecule so as to amplify a desired region thereof. Depending on the length of the probe or primer, the hybridizing nucleic acid probes or primers according to the invention may have at least 90%, 95%, 97%, 98%, or at least 99% complementarity with the segment of the sequence to which they hybridize. In a preferred embodiment, the oligonucleotides according to the invention hybridize to a region within SEQ ID NO:3 and contain from 10 to 30 nucleotides. Nucleic acids having a region of substantial identity to the provided sequences, e. g. allelic variants, genetically-altered versions of the gene, etc., bind to the provided sequences under stringent hybridization conditions. The hybridization procedure is described in Sambrook et al. MOLECULAR CLONING: A LABORATORY MANUAL, 2nd ed., Cold Spring Harbor Laboratory Press, Cold Spring Harbor, N.Y., 1989.

In a further aspect the invention provides antibodies that specifically bind to the subject fluorescent proteins. Suitable antibodies are obtained by immunizing a host animal such as mouse, rat, sheep, goat, hamster, rabbit, etc., with a portion or the entire fluorescent protein of the invention. The immunogen may comprise the complete protein, fragments (e.g. peptides) or derivatives thereof. Antibodies may be monoclonal, polyclonal, single chain or multimeric, immunoglobulin fragments such as Fab, Fc scFv, and may be produced by conventional techniques.

A further aspect of the invention relates to expression vectors containing the indicated nucleic acid molecules. Suitable vectors include viral and non-viral vectors, preferably plasmids, which can be used for cloning, amplifying, expressing or transferring the nucleic acid sequence into an appropriate host.

In a further aspect, the invention provides a prokaryotic or eukaryotic host cell comprising a nucleic acid of the invention or a vector thereof. Host-cells such as *E. coli, B. subtilis, S. cerevisiae,* insect cells, or cells of higher organisms such as vertebrates, particularly COS-7 cells, HEK-293, CHO-K1, Xenopus oocytes, may be used for production of the protein or in cell-based assays. For adequate expression in mammalian cells, the nucleic acid sequences according to the invention may require optimisation of the codon usage. For example, the nucleic acid molecule encoding the fluorescent protein SEQ ID NO:1 may be optimized for mammalian codon usage according to SEQ ID NO:4, which represents a further embodiment of the invention.

The invention further provides transgenic organisms, the genomes of which contain a nucleic acid molecule according to the invention. Transgenic organisms can be prokaryotic or eukaryotic organisms including bacteria, cyanobacteria, fungi, plants and animals, in which one or more of the cells of the organism contain heterologous nucleic acids of the invention. The invention also includes non-human embryonic stem (ES) cells containing a nucleic acid herein disclosed. ES cells grown on an appropriate fibroblastfeeder layer or in the presence of leukemia inhibiting factor (LIF) or other suitable growth factor(s) can be used to produce non-human transgenic animals, including non-human mammals, birds or amphibians, as previously reported. Transgenic plants may also be produced, as reviewed in Plant Biochemistry and Molecular Biology (eds. Lea and Leegood, John Wiley & Sons) (1993) pp. 275-295 and in Plant Biotechnology and Transgenic Plants (eds. Oksman-Caldentey and Barz), (2002) 719 p.

Another aspect of the invention relates to an isolated fluorescent protein which is selected from the group consisting of:
(a) a protein having amino acid sequence SEQ ID NO: 1;
(b) a protein displaying at least 80%, preferably at least 90%, more preferably at least 95% sequence identity to SEQ ID NO: 1;
(c) a fragment of a protein according to (a) or (b), which is water soluble and which retains the capability of emitting green fluorescence upon excitation with light in the range of λ 460-495 nm.

Apart from the indicated sequences which confer the desired fluorescence activity, the amino acid sequences can be modified without negatively affecting the fluorescent protein's activity, especially by conservative substitutions of amino acidic residues within the indicated sequence identity limits. In addition, the protein sequences can be deleted of small portions, without altering the fluorescent activity.

In a preferred embodiment, the fluorescent proteins according to the invention are isolated from organisms of the species *Haeckelia beehleri.* These proteins, which show very low sequence homology to known proteins, are water-soluble and temperature-stable, can be induced in bacteria at 37°C within 3 hours and, thanks to their low bulkiness, are particular suitable for translocation assays.

In a further aspect, the invention provides a method for producing a fluorescent protein, which comprises the steps of:
(a) providing a nucleic acid molecule according to the invention operably linked to expression regulatory elements;
(b) introducing the nucleic acid of step (a) in a cell under conditions permissive for its expression;
(c) isolating the expression product.

Another aspect of the invention is a method for producing green fluorescence in a cell, which comprises:
(a) providing a cell expressing a fluorescent protein according to the invention;
(b) exposing the cells to excitation with light in the range of λ 460-495 nm.

In general, the fluorescent proteins according to the invention or their coding sequences can be used as fluorescent markers, particularly for labelling, localizing or detecting a biological molecule or a compound in a cell or cell organelle, e.g. in a cell-based assay, or they can be used for in vitro assays in a cell-free preparation, which is preferably an aqueous solution.

In typical applications, the fluorescent proteins are used as a tool for the automated screening of arrays of cells expressing fluorescent reporting groups, or as markers fused to peptides (such as targeting sequences) or proteins to detect their intracellular location, or as indicators for cellular activity e.g. in the study of signal transduction. In particular the proteins of the invention can be fused to specific domains such as the PKCgamma Ca-binding domain, PKCgamma DAG binding domain, SH2 domain or SH3 domain to detect the second messengers involved in signalling pathways (Chudakov DM, et al, 2005).

In a preferred embodiment, the cell-based assays involving the fluorescent proteins of the invention are carried out in a high-throughput format utilizing an optical screening tool or apparatus suited for multi-sample analysis or by using microscopic imaging and electronic analysis.

In a further embodiment, the subject proteins are used in the screening for drug discovery and in the field of functional genomics as markers of whole cells to detect changes in multicellular reorganization and migration.

The subject fluorescent proteins also find use in protease cleavage assays as described in US 7,049,400.

In a further embodiment, the proteins of the present invention may be used in high content screening to detect co-localization of other fluorescent fusion proteins with localization markers as indicators of movements of intracellular fluorescent proteins/peptides or as markers alone.

Another application of the subject fluorescent proteins is their use as biosensors in prokaryotic and eukaryotic cells, such as Ca²⁺ ion indicators, pH indicators, phosphorylation indicators or indicators of other ions, such as magnesium, sodium, potassium, chloride and halides (Chudakov DM, et al, 2005).

Secreted forms of the subject proteins, which can be used in a variety of different applications, can be prepared by fusing secretion leader sequences thereto.

In a preferred embodiment, the fluorescent proteins are used as in vivo labels (or reporter molecules) in cell and molecular biology assays, especially in assays for gene expression, protein localization and co-localization, protein-protein interactions, protein-nucleic acid interactions, nucleic acid-nucleic acid interactions, cell and cell organelle localization and interactions.

In a further preferred embodiment, the fluorescent proteins of the invention are used for detecting the effects of a test substance on the regulation of expression and/or translocation of one or more proteins of interest in a cell, or for detecting the expression of a protein of interest and the simultaneous activity of an expression control sequence in response to a test substance. The activity of two or more expression control sequences in a cell in response to a test substance may be compared by using the fluorescent proteins of the invention. Such methods may be performed in the presence and in the absence of a test substance whose effect on the process is to be measured.

In a further embodiment, the fluorescent proteins of the invention are used in fluorescence activated cell sorting (FACS) as markers of cells which can then be sorted with a fluorescent activated cell sorting device.

In addition, the subject proteins may be used in fluorescence resonance energy transfer (FRET) methods as donors and/or acceptors in combination with a second fluorescent protein or dye. Specific examples of FRET assays employing the subject fluorescent proteins include the detection of protein-protein interactions, such as in a mammalian two-hybrid system, transcription factor dimerization, membrane protein multimerization, multiprotein complex formation.

Another application in which the subject fluorescent protein finds use is BRET (Bioluminescence Resonance Energy Transfer). BRET is a protein-protein interaction assay based on energy transfer from a bioluminescent donor to a fluorescent acceptor protein. The BRET signal is measured by the amount of light emitted by the acceptor to the amount of light emitted by the donor. The ratio of these two values increases as the two proteins are brought into proximity.

The fluorescent protein can also be used as a fluorescent timer, in which the switch of one fluorescent colour to another (e. g. green to red) concomitant with the ageing of the fluorescent protein allows to determine the activation/deactivation of gene expression.

In a further embodiment the use of fluorescent proteins in combination with optical imaging procedures improves the knowledge of disease processes and permits a more efficient evaluation of drug *effects in vivo* allowing new diagnostic and therapeutic applications in human medicine. In particular the field of molecular imaging allows both visualization and quantification of molecular events associated with disease in a non-invasive and radiation-free manner using relatively simple equipment (Licha K, et al, 2005 and Weissleder, 2002).

In a yet further aspect, the invention provides a kit suitable for setting up and carrying out an assay or method according to the invention. The kit typically contains a protein or a nucleic acid according to the invention, or it may contain a vector and/or a preparation of cells expressing a fluorescent protein of the invention under the control of a stable or inducible promoter, and reagents suitable for running the assay. The kit may include the cDNA and the oligonucleotide primers able to produce the nucleic acid by PCR. The kit components are typically present in a suitable storage medium, such as a buffered solution, packaged in a suitable container. The kit may also include antibodies specific to the provided protein.

The invention will be illustrated in greater detail in the following experimental section.

### MATERIALS AND METHODS

### cDNA library construction

The Creator^{™} SMART^{™} cDNA Library Construction Kit (Clontech Laboratories, Inc., Mountain View, CA) was used, following manufacturers instructions. It provides a method for producing high-quality, full-length cDNA libraries from nanograms of total RNA.

### cDNA synthesis by Long-Distance PCR

Library construction employs long-distance PCR (LD PCR; Barnes WM et al., 1994, Cheng S et al., 1994) for generating full-length cDNA. This protocol is ideal when working with small amounts of RNA (i.e. 50 ng of total RNA). A modified oligo(dT) primer (CDS III/3' PCR Primer) primes the first-strand synthesis reaction, and the SMART IV Oligo serves as a short, extended template at the 5' end of the mRNA When the RT reaches the 5' end, the enzyme's terminal transferase activity adds a few additional nucleotides, primarily deoxycytidine, to the 3' end of the cDNA. The SMART IV Oligo, which has an oligo(G) sequence at its 3' end, base-pairs with the deoxycytidine stretch, creating an extended template. RT then switches templates and continues replicating to the end of the oligonucleotide.

The resulting full-length ss cDNA contains the complete 5' end of the mRNA, as well as the sequence complementary to the SMART IV Oligo, which then serves as a universal priming site (SMART anchor) in the subsequent amplification by LD PCR. For LD PCR reaction specific primers have been designed, based on the SMART IV oligo and CDS III/3' PCR Primer sequences but having at their ends respectively a *Sal* I and a *Not* I restriction site for subsequent cloning into the chosen vector (specific primer sequences are below).

### Primers

### SMART IV/LD Sal I

Sequence: ACGCGTCGACGTAAGCAGTGGTATCAACGCAGAGT

### CDS III/LD Not I

Sequence:
AAGGAAGAGCGGCCGCATTCTAGAGGCCGAGGCGGCCGAC

Amplification protocol performed using the Perkin Elmer 2400 thermocycler:
1 time the following step:
   pre PCR 20" at 95°C
22 times the following steps:
   denaturation 5" at 95°C
   annealing and elongation 6' at 68°C

### Sall / NotI digestion

The cDNAs were digested for 2 hours at 37°C with the restriction enzymes *Sal* I and *Not* I.

### Column chromatography

The SuperScript^{™} System for cDNA Libraries was used in the subsequent steps of the library construction following manufacturer's indications (Invitrogen Corp., Carlsbad, CA.). The recommended procedure of cDNA size fractionation by column chromatography allowed the selection of fractions enriched for large cDNAs, while small inserts (< 500bp) (partially degraded mRNAs or incompletely synthesized mRNAs), which usually dominate the library, were excluded.

### Plasmid pSPORT 1

The pSPORT 1 plasmid belonging to the SuperScript^{™} System for cDNA Libraries (Invitrogen Corp., Carlsbad, CA.) was used.

### Expression of cloned cDNA

Plasmid pSPORT 1 can be used to express cloned genes by inducing the *lac* promoter (*lac*P). The vector contains a single copy of the *lac* repressor gene (*lac*I); transcription of cDNA inserts from the promoter is effectively repressed unless the promoter is induced by the addition of 1 mM isopropylthio-β-galactoside (IPTG) to plates or liquid media. Libraries are not plated under inducing conditions unless expression is specifically desired. Inducing expression can lead to loss of clones from the library due to accumulation of proteins that may interfere with the metabolism *of E. coli.*

### Ligation of cDNA to the vector

The ligation reaction using the SuperScript^{™} System for cDNA Libraries was performed as indicated by the manufacturer (Invitrogen Corp., Carlsbad, CA.).

### Bacterial strain

For good protein expression we chose BL21-Gold(DE3) cells (Stratagene, La Jolla, CA), a derivative of *E. coli* B, an improved strain of BL21 competent cells. The genotype of the strain is: *E. coli* B F⁻ *ompT hsdS* (r_{B}⁻ m_{B}⁻) *dcm⁺* Tet^{r} *gal* λ(DE3) *endA* Hte.

In order to obtain competent cells with a high efficiency of transformation, we followed the standard protocol for preparing and electro-transforming BL21-Gold(DE3) cells described in the *E. coli* Pulser Transformation apparatus manual (BioRad).

Transformation efficiency was tested by using pUC18 DNA and the pET DNA vectors and the efficiencies obtained were:
- 1x10¹⁰ cfu/µg of pUC18 DNA
- 1x10⁸ cfu/µg of pET DNA

Using these highly electrocompetent cells, we were able to obtain a library of approximately 15,000 colonies expressing the cDNA library.

### Transformation, plating and induction

Transformed bacterial cells were plated on an LB agar plates and grown overnight at 37°C. After overnight colony growth, induction was obtained by adding 2 mM IPTG and incubating for 3 hours at RT.

### Functional screening

About 15,000 recombinant clones were screened visually using a fluorescent microscope. One fluorescent clone encoding for a green fluorescent protein was identified, picked and plated on a new LB agar plate.

### Induction of protein expression in liquid and analysis on SDS-PAGE gels under denaturing and non-denaturing conditions

Eight single colonies, all coming from the originally identified green fluorescent clone, were grown in 5 ml of LB liquid medium O/N at 37°C. On the following day, a diluted aliquot of each bacterial culture was grown for two hours at 37°C and then induced with 1 mM IPTG at 37°C for 3 hours. Bacterial lysis followed by centrifugation was performed to obtain two different fractions for each sample: the pellet (not soluble) and the supernatant (soluble). Samples of each fraction were loaded on SDS-PAGE both in denaturing and non-denaturing conditions.

### GFP optimization for expression in mammalian cells (GENEART AG Regensburg, Germany)

The codon usage of the wild-type gene was adapted to the codon bias of highly expressed mammalian genes. In addition regions of very high (> 80%) or very low (< 30%) GC content have been avoided wherever possible. For efficient translation initiation, the Kozak-consensus sequence was introduced upstream of the start codon. One additional STOP codon was added to the existing one to ensure efficient termination. The corresponding optimized gene was assembled from synthetic oligonuleotides. The fragment was cloned into pGA4 plasmid using *Kpn* I and *Sac* I restriction sites. The final construct was verified by sequencing.

### DESCRIPTION OF THE DRAWINGS

**Fig. 1:** Bacterial colony expressing HbGFP.
**Fig. 2:** Protein gel-electrophoresis analysis of HbGFP (denaturing conditions). Comparison of the mobility of not-induced, induced (37°C), pellet and supernatant fractions.
**Fig. 3**: Pseudonative gel analysis of induced bacterial cultures of HbGFP (supernatant fractions). Comparison of the mobility of heated versus non-heated protein samples.

### EXAMPLES

### 1. Expression library construction and screening

To identify the protein responsible for fluorescence in this Ctenophora, a strategy based on the screening of an expression library in *E. coli* was chosen. Amplified cDNAs were prepared using a SMART cDNA amplification kit (Clontech Laboratories, Inc., Mountain View, CA) and cloned into pSPORT 1 vector (Invitrogen Corp., Carlsbad, CA.).

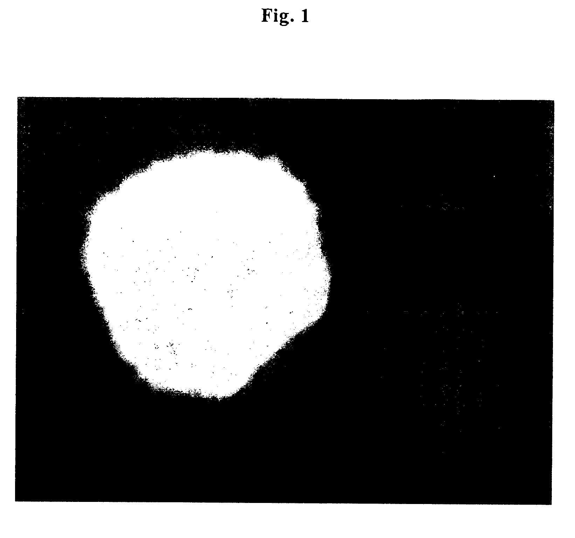

A total of 15,000 colonies were screened visually using a fluorescent microscope. One positive colony was obtained (Fig. 1) and named HbGFP. The amino acidic and nucleic acid sequences of the fluorescent protein (named HbGFP) isolated therefrom are shown in SEQ NOs: 1 and 2, respectively. Comparison of HbGFP with *A. victoria* GFP and *A. sulcata* GFP shows 26% and 24% amino acidic identity, respectively.

### 2. Haeckelia beehleri fluorescent protein characterization

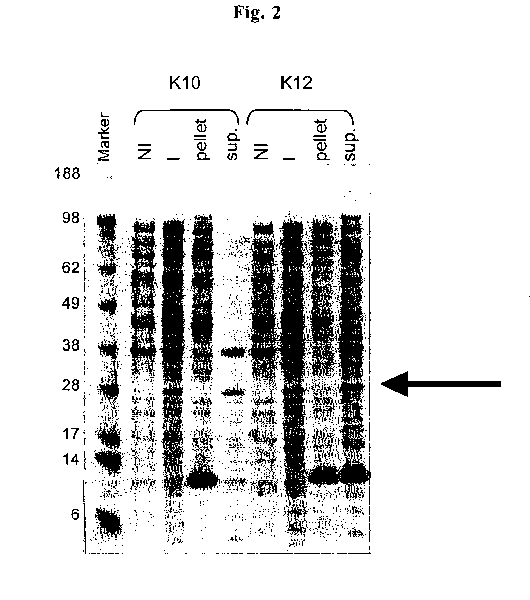

In order to characterize the new fluorescent protein, expression was induced in *E*. *coli* and then analyzed on SDS gels both in denaturing and non-denaturing conditions. Eight single colonies, all coming from the originally identified green fluorescent clone, were grown in LB liquid medium and then induced with 1 mM IPTG at 37°C for 3 hours. Bacterial lysis followed by centrifugation was performed to obtain two different fractions for each sample: the pellet (not soluble) and the supernatant (soluble). Aliquots of each fraction were loaded on SDS-PAGE in denaturing conditions (**Fig. 2**). A band of the predicted molecular weight (~30 kDa) was present in the induced as well as in the soluble samples, thus indicating that the protein is well expressed at 37°C after a short induction and that it is soluble.

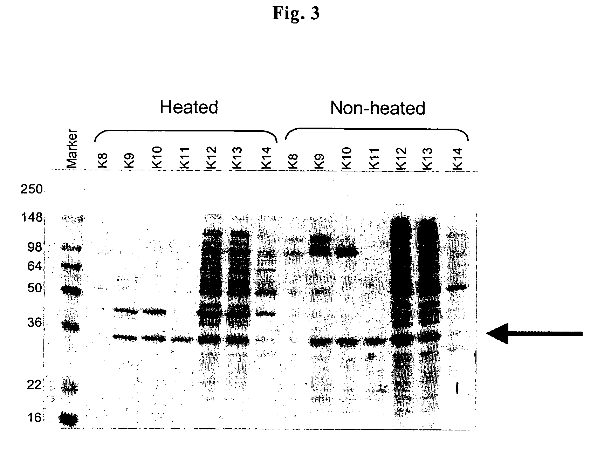

GFP-like proteins retain their spectral properties and oligomerization state under conditions of pseudonative gel electrophoresis, where the samples are not heated before being loaded onto the gel (Baird GS et al., 2000). This test was used to examine the folding state of HbGFP. Aliquots of each soluble fraction were loaded on SDS-PAGE, comparing the mobility of heated versus non-heated samples. In both the heated (denatured) and the not-heated (non-denatured) samples, HbGFP runs as a uniform band of -30 kDa, thus indicating a monomeric structure (**Fig. 3**).

### 3. Expression of the fluorescent protein in mammalian cells Reagents

Restriction enzymes were purchased from New England Biolabs (Beverly, MA) and used according to supplier's instructions. Rapid DNA ligation kit and FuGENE® 6 Transfection Reagent were purchased from Roche (Basel, CH). All other chemicals were from standard sources and were of reagent grade or better.

### Cloning procedure

For fluorescent labelling of eukaryotic cells, the humanized version of HbGFP was cloned into pEGFP-N1 vector (Clontech Laboratories, Inc., Mountain View, CA) between *Bam*H I and *Not* I restriction sites (in lieu of the EGFP-coding region) as well as in other mammalian expression vectors.

### Transfection

CHO-K1, HEK-293, HeLa cell lines were used for the expression of the humanized fluorescent protein in eukaryotic system. Cells were transfected using FuGENE® 6 Reagent and tested by FACS and using a fluorescent microscope both for transient and stable expression. Fluorescence was clearly detectable 16 hours after transfection, it was maintained and no cell toxicity was observed.

### 4. Recombinant protein production

For the production of the recombinant protein we followed a small scale protein purification protocol under native conditions. Due to the presence of an N-terminal His tag in our constructs, we decided to purify the expressed proteins on Ni-NTA Spin Columns (Qiagen) following the suppliers' protocol.

### REFERENCES

Baird GS, Zacharias, DA and Tsien, RY (2000) Biochemistry, mutagenesis and oligomerization of DsRed, a red fluorescent protein from coral. Proc. Natl. Acad. Sci. U.S.A. 97, 11984-11989.
Barnes WM.; PCR amplification of up to 35-kb DNA with high fidelity and high yield from lambda bacteriophage templates. Proc Natl Acad Sci U S A. 1994;91(6):2216-20.
Chalfie M, Tu Y, Euskirchen G, Ward WW, Prasher DC. Green fluorescent protein as a marker for gene expression. Science. 1994 Feb 11;263(5148): 802-5.
Cheng S, Fockler C, Barnes WM, Higuchi R; Proc. Effective amplification of long targets from cloned inserts and human genomic DNA. Natl Acad Sci USA. 1994; 91(12):5695-9.
Chudakov DM, Lukyanov S and Lukyanov KA; Fluorescent proteins as a toolkit for in vivo imaging. Trends in Biotechnology. 2005 Dec, 23 (12): 605-613.
Cody CW, Prasher DC, Westler WM, Prendergast FG, Ward WW. Chemical structure of the hexapeptide chromophore of the Aequorea green-fluorescent protein. Biochemistry. 1993 Feb 9;32(5): 1212-8.
Cormier MJ, Hori K, Karkhanis YD, Anderson JM, Wampler JE, Morin JG, Hastings JW.; Evidence for similar biochemical requirements for bioluminescence among the coelenterates. J Cell Physiol. 1973 Apr;81(2): 291-7.
Cormier MJ, Hori K, Anderson JM.; Bioluminescence in coelenterates. Biochim Biophys Acta. 1974 Oct 31;346(2):137-64.
Davenport D, Nichol JAC. Luminescence in Hydromedusae. Proceedings of the Royal Society, Series B 1955, 144:399-411.
Fradkov AF, Chen Y, Ding L, Barsova EV, Matz MV, Lukyanov SA. Novel fluorescent protein from Discosoma coral and its mutants possesses a unique far-red fluorescence. FEBS Lett. 2000 Aug 18;479(3):127-30.
Gurskaya NG, Fradkov AF, Terskikh A, Matz MV, Labas YA, Martynov VI, Yanushevich YG, Lukyanov KA, Lukyanov SA. GFP-like chromoproteins as a source of far-red fluorescent proteins. FEBS Lett. 2001 Oct 19;507(1):16-20.
Heim R, Prasher DC, Tsien RY. Wavelength mutations and posttranslational autoxidation of green fluorescent protein. Proc Natl Acad Sci U S A. 1994 Dec 20;91(26):12501-4.
Herring, PJ; How to survive in the dark: bioluminescence in the deep sea, Symp Soc Exp Biol. 1985;39:323-50.
Johnson, FH, Gershman LC, Waters JR, Reynolds GT., Saiga Y, and Shimomura O. Quantum efficiency of Cypridina luminescence, with a note that of Aequorea. J. Cell. Comp. Physiol. 1962. 60:85-104.
Kendall JM, Badminton MN. Aequorea victoria bioluminescence moves into an exciting new era.Trends Biotechnol. 1998 May;16(5):216-24.
Labas YA, Gurskaya NG, Yanushevich YG, Fradkov AF, Lukyanov KA, Lukyanov SA, Matz MV. Diversity and evolution of the green fluorescent protein family. Proc Natl Acad Sci U S A. 2002 Apr 2;99(7):4256-61.
Licha K, Olbrich C. Optical imaging in drug discovery and diagnostic applications. Adv Drug Deliv Rev. 2005 Jun 15;57(8):1087-108.
Lukyanov KA, Fradkov AF, Gurskaya NG, Matz MV, Labas YA, Savitsky AP, Markelov ML, Zaraisky AG, Zhao X, Fang Y, Tan W, Lukyanov SA. Natural animal coloration can Be determined by a nonfluorescent green fluorescent protein homolog. J Biol Chem. 2000 Aug 25;275(34):25879-82.
Matz MV, Fradkov AF, Labas YA, Savitsky AP, Zaraisky AG, Markelov ML, Lukyanov SA. Fluorescent proteins from nonbioluminescent Anthozoa species. Nat Biotechnol. 1999 Oct;17(10):969-73.
Morin JG, Hastings JW. Energy transfer in a bioluminescent system. J Cell Physiol. 1971 Jun;77(3):313-8.
Morin JG, Reynolds GT. The cellular origin of bioluminescence in the colonial hydroid Obelia. Biol Bull. 1974 Oct;147(2):397-410.
Morise H, Shimomura O, Johnson FH, Winant J., Intermolecular energy transfer in the bioluminecent system of Aequorea. Biochemistry 1974, 13:2656-2662.
Ormo M, Cubitt AB, Kallio K, Gross LA, Tsien RY, Remington SJ. Crystal structure of the Aequorea victoria green fluorescent protein. Science. 1996 Sep 6;273(5280):1392-5.
Prasher DC, Eckenrode VK, Ward WW, Prendergast FG, Cormier MJ. Primary structure of the Aequorea victoria green-fluorescent protein. Gene. 1992 Feb 15;111 (2):229-33.
Prendergast FG, Mann KG. Chemical and physical properties of aequorin and the green fluorescent protein isolated from Aequorea forskalea. Biochemistry. 1978 Aug 22;17(17):3448-53.
Shimomura O, Johnson FH, Saiga Y.; Extraction, purification and properties of aequorin, a bioluminescent protein from the luminous hydromedusan, Aequorea. J Cell Comp Physiol. 1962 Jun;59:223-39.
Tsien RY. The green fluorescent protein. Annu Rev Biochem. 1998;67: 509-44.
Verkhusha VV, Lukyanov KA. The molecular properties and applications of Anthozoa fluorescent proteins and chromoproteins. Nat Biotechnol. 2004 Mar;22(3):289-96.
Wampler JE, Hori K, Lee JW, Cormier MJ.; Structured bioluminescence. Two emitters during both the in vitro and the in vivo bioluminescence of the sea pansy, Renilla. Biochemistry. 1971 Jul 20;10(15):2903-9.
Weissleder R. Scaling down imaging: molecular mapping of cancer in mice. Nat Rev Cancer. 2002 Jan; 2(1):11-8.
Wiedenmann J, Elke C, Spindler KD, Funke W. Cracks in the beta-can: fluorescent proteins from Anemonia sulcata (Anthozoa, Actinaria). Proc Natl Acad Sci U S A. 2000 Dec 19;97(26):14091-6.
Yang F, Moss LG, Phillips GN Jr. The molecular structure of green fluorescent protein. Nat Biotechnol. 1996 Oct;14(10):1246-51.

## Claims

1. An isolated nucleic acid molecule encoding a fluorescent protein or polypeptide selected from the group consisting of:
(a) a protein having amino acid sequence SEQ ID NO:1;
(b) a protein displaying at least 80%, preferably at least 90%, more preferably at least 95% sequence identity to SEQ ID NO: 1;
(c) a fragment of a protein according to (a) or (b), which is water-soluble and which retains the capability of emitting green light upon excitation with light in the range of λ 460-495 nm.

2. A nucleic acid molecule according to claim 1, which is selected from the group consisting of:
(a) a nucleic acid comprising the nucleotide sequence SEQ ID NO:2 or SEQ ID NO:3 or a complementary strand thereof;
(b) a nucleic acid differing from SEQ ID NO:2 due to the degeneracy of genetic code;
(c) a nucleic acid that hybridizes under stringent conditions to the nucleic acid of (a) or (b).

3. A nucleic acid molecule according to claim 2, which has the sequence SEQ ID NO:4.

4. A nucleic acid molecule according to claim 2, which is isolated from organisms belonging to *Haeckelia beehleri* species.

5. An oligonucleotide hybridizing to the nucleic acid molecule of claim 1 or 2.

6. An oligonucleotide according to claim 5, hybridizing to a region within SEQ ID NO:3 and containing from 10 to 30 nucleotides.

7. A eukaryotic or prokaryotic expression vector containing a nucleic acid according to claims 1-4.

8. A prokaryotic or eukaryotic host cell comprising a nucleic acid according to claims 1-4 or a vector according to claim 7.

9. The host-cell of claim 8, which is a *E. coli, B. subtilis, S. cerevisiae,* COS-7, HEK- 293, CHO-K1 cell, an insect cell or a Xenopus oocyte.

10. An isolated fluorescent protein or polypeptide which is selected from the group consisting of:
(a) a protein having amino acid sequence SEQ ID NO: 1;
(b) a protein displaying at least 80%, preferably at least 90%, more preferably at least 95% sequence identity to SEQ ID NO: 1;
(c) a fragment of a protein according to (a) or (b), which is water-soluble and which retains the capability of emitting green light in response to excitation with light in the range of λ 460-495 nm.

11. A protein according to claim 10, which is isolated from organisms of the species *Haeckelia beehleri.*

12. A monoclonal or polyclonal antibody, or a fragment thereof, able to specifically bind the fluorescent protein of claim 10 or 11.

13. A method for producing a fluorescent protein, which comprises the steps of:
(a) providing a nucleic acid molecule according to claims 1-4 operably linked to expression regulatory elements;
(b) introducing the nucleic acid of step (a) into a cell under conditions permissive for its expression;
(c) isolating the expression product.

14. A method for producing green fluorescence in a cell, which comprises:
(a) providing a cell expressing a fluorescent protein according to claims 10 or 11;
(b) exposing the cells to light excitation in the range of λ 460-495 nm.

15. The use of a protein according to claims 10-11 or of a nucleic acid according to claims 1-4, as a fluorescent marker.

16. The use according to claim 15, for labelling, localizing or detecting a biological molecule in a cell or cell organelle.

17. The use according to claim 16, in a translocation assay.

18. A cell-based assay utilizing a protein according to claims 10-11 as an intracellular fluorescent label.

19. A cell-based assay according to claim 18, which is carried out in a high-throughput format utilizing an optical screening tool or apparatus suited for multi-sample analysis or by using microscopic imaging and electronic analysis.

20. A kit for carrying out the method of claims 13-14 or the assay of claim 18, which comprises a protein according to claims 10-11 and/or a nucleic acid according to claims 1-4 and/or an oligonucleotide according to claims 5-6.

21. A cell-free preparation containing a protein according to claims 10-11.

22. A preparation according to claim 19, which is a water solution.
